# EUROPÄISCHE PATENTSCHRIFT

(11) **EP 1 646 315 B1**
(45) Veröffentlichungstag und Bekanntmachung des Hinweises auf die Patenterteilung: **29.10.2014**
(21) Anmeldenummer: 04762330.1
(22) Anmeldetag: 02.07.2004
(51) Int. Cl.: A61B 5/00

(54) **VORRICHTUNG ZUR INDUKTION VON NICHTGEWEBSSCHÄDIGENDEN REIZEN ZUM TESTEN EINER MECHANISCH INDUZIERTEN SCHMERZEMPFINDUNG EINES PROBANDEN**
DEVICE FOR INDUCING NON-TISSUE DAMAGING STIMULI IN ORDER TO TEST A MECHANICALLY INDUCED SENSATION OF PAIN IN A TEST PERSON
DISPOSITIF SERVANT A INDUIRE DES STIMULI N'ENTRAINANT PAS DE DOMMAGE TISSULAIRE, POUR VERIFIER UNE SENSATION DE DOULEUR INDUITE MECANIQUEMENT CHEZ UN SUJET D'EXPERIENCE

(30) Priorität: 10.07.2003 DE 10331250
(43) Veröffentlichungstag der Anmeldung: 19.04.2006
(73) Patentinhaber: Johannes Gutenberg-Universität Mainz, 55122 Mainz (DE)
(72) Erfinder: MAGERL, Walter, 55116 Mainz (DE); TREEDE, Rolf-Detlef, 55127 Mainz (DE)
(74) Vertreter: Müller, Jochen
(86) Internationale Anmeldenummer: PCT/DE2004/001404
(87) Internationale Veröffentlichungsnummer: WO 2005/006982

(56) Entgegenhaltungen:
- WO-A-99/07279
- DE-A- 10 126 690
- US-A- 4 964 412
- US-A- 5 810 743
- US-B1- 6 234 977

## Beschreibung

Die Erfindung bezieht sich auf eine Vorrichtung zur Induktion von nichtgewebsschädigenden Reizen zum Testen einer mechanisch induzierten Schmerzempfindung eines Probanden mit einem Griffstück, in dem eine von einer definierten Kraft beaufschlagte Nadel mit abgeflachter Spitze gleitbeweglich gelagert ist.

Eine Vorrichtung zur Induktion von nichtgewebsschädigenden Reizen zum Testen einer mechanisch induzierten Schmerzempfindung eines Probanden mit einem Griffstück, dem eine Nadel mit abgeflachter Spitze zugeordnet ist, wobei die Nadel gleitbeweglich in dem Griffstück gelagert und von einer definierten Kraft durch eine Feder beaufschlagt ist, ist jeweils aus der DE 101 26 690 A1, US-A-4 964 412 und WO 99/07279 A bekannt.

Die Messung der Schmerzempfindung eines Probanden erfordert die Anwendung von neurophysiologisch aus der Arbeitsweise der Nozisensoren, also der Sensoren des Schmerzsystems, abgeleitete reizauslösende Vorrichtungen, wobei die Nozisensoren durch aktuell oder potentiell gewebsschädigende Reize, beispielsweise durch Schnitt, Stich oder Quetschung, aktiviert werden.

Die DE 693 29 419 T2 offenbart einen neurologischen Stift mit einem Griffstück, dessen eines Ende scharf und dessen anderes Ende stumpf ausgebildet ist, wobei beide Ende zum Testen einer objektiven Empfindung bestimmt sind. Das scharfe Ende ist von einem eine ringförmige Fläche definierenden Element umgeben, das das Ausmaß bestimmt, in dem das scharfe Ende in die Haut eines Probanden unter einer bestimmten Kraft eindringen kann. Hierbei erweist es sich als problematisch, dass durch ein Anpressen der ringförmigen Fläche bei einer erhöhten Kraftbeaufschlagung des Stiftes das Testergebnis verfälscht sein kann, da der Proband nicht den stechenden Schmerz des scharfen Endes sondern den stumpfen Schmerz des das scharfe Ende umgebenden Elementes wahrnimmt und rückmeldet. Diese Gefahr besteht insbesondere bei einem Probanden mit einem verhältnismäßig dicken Fettgewebe oder einer relativ weichen Haut, da unter diesen Gegebenheiten das spitze Ende ohne die Auslösung eines Schmerzes versinkt.

Es ist Aufgabe der Erfindung, eine Vorrichtung der eingangs genannten Art zu schaffen, mit der nadelstichähnliche Reize ohne eine Schädigung des Körpergewebes reproduzierbar ausgelöst werden und die dabei einen einfachen Aufbau aufweist.

Erfindungsgemäß wird die Aufgabe dadurch gelöst, dass die Nadel auswechselbar an einem in dem Griffstück verschiebbar gelagerten Kolben definierter Masse befestigt ist, wobei aus der Masse des Kolbens die definierte Kraft resultiert.

Aufgrund dieser Maßnahmen eignet sich die Vorrichtung zur Bestimmung der individuellen mechanisch induzierten Schmerzschwelle und der überschwelligen Reiz-Antwortfunktion eines Probanden ohne eine Schädigung des Körpergewebes, da die Kraft entsprechend bemessen ist und die Nadel den Probanden unabhängig von der auf das Griffstück ausgeübten Anpresskraft lediglich mit der ihr zugeordneten Masse beaufschlagt und nahezu reibungsfrei in das Griffstück eintaucht. Zur Untersuchung von Zuständen veränderter Schmerzempfindung sowie therapiebegleitender Veränderungen der Schmerzempfindung eines Probanden bzw. Patienten und zur reproduzierbaren quantitativen Beschreibung der Schmerzempfindung werden mehrere Vorrichtungen eingesetzt, deren Nadeln mit unterschiedlichen Kräften, beispielsweise mit 8, 16, 32, 64, 128, 256 und 516 mN, beaufschlagt sind. Die unterschiedlichen Krafteinwirkungen bilden entsprechend den Grundprinzipien der somatosensorischen Wahrnehmung eine geometrische Reihe und umfassen einen Bereich unterhalb, um und oberhalb der Schmerzempfindungsschwelle, wobei die größte verwendete Kraft so bemessen ist, dass sie eine Verletzung der Haut ausschließt. Die Abflachung der Nadel bzw. deren den Probanden berührende freie Stirnfläche und deren Durchmesser sind derart gestaltet bzw. bemessen, dass eine Verschiebung des Körpergewebes von der Oberfläche in die Tiefe erfolgt, womit eine lokale Scherung des Gewebes ohne Schädigung der Gewebsoberfläche einhergeht. Hierzu weist die Nadel beispielsweise einen Durchmesser zwischen 50 und 500 µm, bevorzugt 200 µm auf.

Das auswechselbare Haltern der Nadel trägt Hygieneansprüchen im klinischen Einsatz der Vorrichtung Rechnung. Da der Kolben die definierte Masse aufweist, bewirkt er eine Belastung der Nadel mit einer definierten Kraft. Der Kolben übernimmt darüber hinaus die Führung der Nadel in dem Griffstück. Um die Nadel mit unterschiedlichen Kräften auf das Gewebe des Probanden zu pressen, ist es lediglich erforderlich, Kolben mit zu den Kräften proportionalen Massen bereitzustellen. Vorzugsweise ist die Nadel an der Stirnseite einer Kappe angeordnet, die an einer Stirnseite des Kolbens zu dessen Gleitführung in einer zentrischen Führungsbohrung in dem Griffstück festgelegt ist. Die Kappe stellt zum einen ein Führungsmittel für den Kolben dar, da sie mit ihrem äußeren Umfang in der Führungsbohrung des Griffstückes gleitet und zum anderen dient sie als Halterung für die Nadel.

Um die relativ lange Nadel im Bereich ihrer Halterung zu stabilisieren, ist zweckmäßigerweise die Nadel in eine in der Kappe befestigte Führungshülse eingesetzt.

Bevorzugt weist die im Querschnitt zylindrische Kappe einen Ansatz auf, der aus einer stirnseitigen Öffnung des Griffstückes nach außen ragt. Zum relativ einfachen Entnehmen der Nadel ist der Ansatz mit einer Ringnut versehen. In die Ringnut kann ein Halter oder eine Zange eingesetzt werden, um den Kolben zu fixieren und mit der Hand oder einem geeigneten Werkzeug die Nadel aus der Führungshülse der Kappe zu ziehen.

Für eine zuverlässige Lagerung des Kolbens in der Führungsbohrung des Griffstückes ist der Kolben unter Zwischenanordnung mindestens eines Führungsringes in der Führungsbohrung des Griffstückes geführt. Zur Bewerkstelligung der verschiebbaren Führung des Kolbens in der Führungsbohrung des Griffstückes mit einer geringen Reibung ist die Kappe und/oder der Führungsring aus Polytetrafluorethylen (PTFE) gefertigt.

Damit der Kolben nicht aus dem Griffstück herausgleitet, ist vorteilhafterweise das Griffstück auf der der Nadel gegenüberliegenden Seite mit einem Deckel verschlossen. Zweckmäßigerweise ist der Deckel in das Griffstück eingepresst oder eingeschraubt. Nach dem Entfernen des Deckels kann der Kolben dem Griffstück zu seiner Reinigung entnommen werden.

Es ist zu vermeiden, dass die die Nadel beaufschlagende Kraft eine Veränderung aufgrund der in der Führungsbohrung des Griffstückes eingeschlossenen Luft erfährt, die bei einer Benutzung der Vorrichtung eine in die eine oder andere Richtung wirkende Kraft auf den Kolben auswirkt. Zur Zirkulation der Luft in der Führungsbohrung des Griffstückes weist vorzugsweise der Deckel eine konzentrisch zu dem Kolben ausgerichtete Durchgangsbohrung auf. Zweckmäßigerweise ist in die dem Deckel zugeordnete Stirnseite des Kolbens eine Gewindebohrung fluchtend zu der Durchgangsbohrung des Deckels eingelassen.

Um den Kolben unverlierbar in dem Griffstück zu lagern, weist bevorzugt die Führungsbohrung des Griffstückes stirnseitig einen verjüngenden Absatz auf, der ein Austreten der Kappe aus dem Griffstück verhindert. Lediglich der Ansatz der Kappe gelangt nach außen, da er einen gegenüber dem übrigen Verlauf der Kappe verkleinerten Durchmesser aufweist. Die zwischen dem Ansatz und dem im Griffstück befindlichen Teil der Kappe ausgebildete Schulter kommt in einer unteren Endlage des Kolbens an dem Absatz der Führungsbohrung zur Anlage. Dem Absatz ist innerhalb der Führungsbohrung ein O-Ring zugeordnet, der den Ansatz der Kappe mit Spiel umgreift. Beim Gleiten des Kolbens in seine untere Endlage federt der O-Ring, der aus einem elastomeren Kunststoff gefertigt ist, den Aufprall des Kolbens ab.

Vorteilhafterweise ist die Länge der Nadel derart bemessen, dass sich ihr freies Ende innerhalb der Führungsbohrung befindet, wenn der Kolben an dem Deckel anliegt. Aufgrund der relativ lang bemessenen Nadel ist diese obere Endlage des Kolbens für den Benutzer der Vorrichtung optisch leicht zu erkennen und damit ist ein Aufsetzen des Griffstückes zur undefinierten Induktion eines Schmerzes ausgeschlossen. Des Weiteren ist die maximale Länge der Nadel im Wesentlichen durch den Verschiebeweg des Kolbens und die Länge des Griffstückes begrenzt, weshalb die Nadel so lang ausgeführt sein kann, dass sie auch bei einem Probanden mit einem verhältnismäßig dicken Fettgewebe einen Schmerz auslöst.

Um die Nadel während des Transportes der Vorrichtung vor Beschädigungen zu schützen, ist zweckmäßigerweise als Transportsicherung eine in die Durchgangsbohrung des Deckels eingesetzte Schraube vorgesehen, die in die Gewindebohrung des Kolbens eingreift, um denselben an dem Deckel festzulegen. Sonach ist der Kolben in seiner oberen Endlage gehalten und die Nadel befindet sich innerhalb des Griffstückes.

Damit sich die Vorrichtung bei einer Untersuchung des Probanden mittels eines bildgebenden Verfahrens der medizinischen Diagnostik, beispielsweise der Magnetresonanztomographie, nicht störend auswirkt, sind vorteilhafterweise das Griffstück, der Kolben und/oder die Nadel aus Aluminium, Messing oder einem nicht magnetischen Edelstahl gefertigt.

Es versteht sich, dass die vorstehend genannten und nachstehend noch zu erläuternden Merkmale nicht nur in der jeweils angegebenen Kombination, sondern auch in anderen Kombinationen verwendbar sind. Der Rahmen der vorliegenden Erfindung ist nur durch die Ansprüche definiert.

Die Erfindung wird im Folgenden anhand eines Ausführungsbeispieles unter Bezugnahme auf die zugehörigen Zeichnungen näher erläutert. Es zeigen:

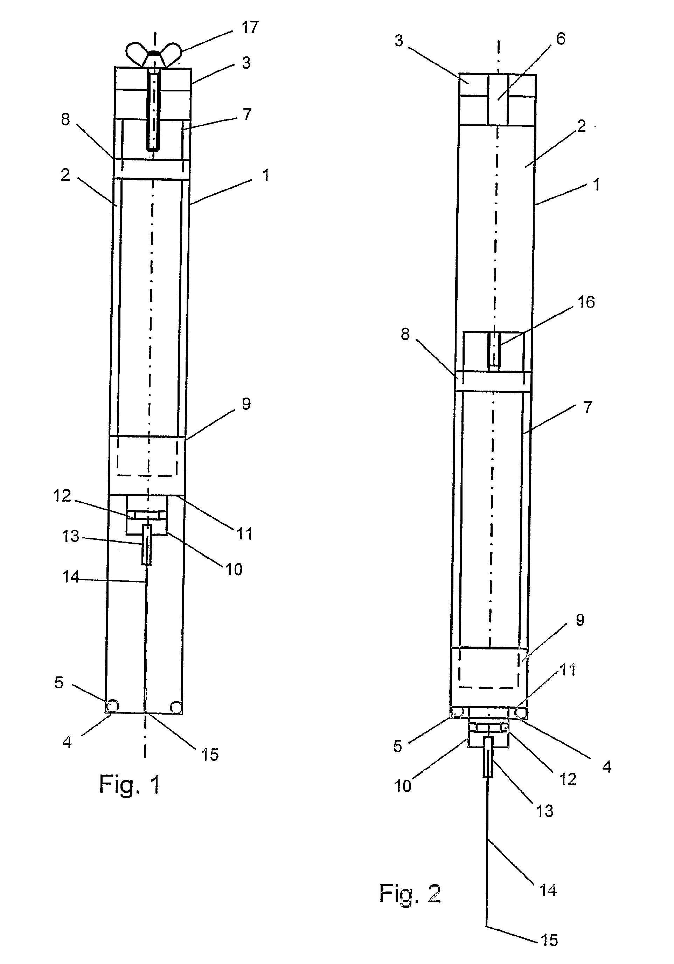

- Fig.1: eine Darstellung der erfindungsgemäßen Vorrichtung im Teilschnitt mit einer wirksamen Transportsicherung und
- Fig.2: eine Darstellung der Vorrichtung gemäß Fig. 1, deren Kolben sich in einer unteren Endlage befindet.

Die Vorrichtung umfasst ein zylindrisches Griffstück 1 mit einer zentrischen Führungsbohrung 2, die an einem Ende mittels eines Deckels 3 verschlossen ist und am anderen Ende einen verjüngenden Absatz 4 mit einem innenliegenden O-Ring 5 aufweist. In den Deckel 3 ist koaxial zur Führungsbohrung 2 eine Durchgangsbohrung 6 eingearbeitet. Innerhalb der Führungsbohrung 2 ist ein Kolben 7 definierter Masse verschiebbar gelagert, wozu er zum einen mit einem zylindrischen Führungsring 8 und zum anderen mit einer Kappe 9 versehen ist, die auf die dem Absatz der Führungsbohrung 2 zugeordneten Stirnseite des Kolbens 7 aufgepresst ist. Die Kappe 9 weist einen Ansatz 10 mit einem gegenüber dem Durchmesser der Kappe 9 reduzierten Durchmesser auf, der sich zumindest in einer unteren Endlage des Kolbens 7 durch den verjüngenden Absatz 4 der Führungsbohrung 2 des Griffstückes 1 und den dem Absatz 4 zugeordneten O-Ring 5 mit Spiel nach außen erstreckt. Eine zwischen dem Ansatz 10 und der Kappe 7 ausgebildete Schulter 11 kommt in der unteren Endlage des Kolbens 7 an dem O-Ring 5 zur Anlage, um die Gleitbewegung des Kolbens 7 zu dämpfen. Umfangsseitig ist in den Ansatz 10 eine Ringnut 12 eingestochen, die sich in der unteren Endlage des Kolbens 7 außerhalb des Griffstückes 1 befindet. In die freie Stirnseite des Ansatzes 10 der Kappe 7 ist eine Führungshülse 13 zur Aufnahme einer Nadel 14, eines so genannten Reizfilamentes, eingesetzt. Die Nadel 14 weist eine derart abgeflachte Spitze 15 auf, dass sich ihre Stirnfläche rechtwinklig zur Umfangsfläche erstreckt.

Zum Transport der Vorrichtung wird der Kolben 7 in seine obere Endlage verschoben, in der seine dem Deckel 3 zugewandte Stirnseite, in die eine koaxial zur Durchgangsbohrung 6 des Deckels 3 ausgerichtete Gewindebohrung 16 eingearbeitet ist, an dem Deckel 3 anliegt. Durch die Durchgangsbohrung 6 im Deckel 3 wird eine Schraube 17 in die Gewindebohrung 16 des Kolbens 7 eingeschraubt, wodurch der Kolben in seiner oberen Endlage fixiert ist, in der sich die Spitze 15 der Nadel 14 geringfügig beabstandet zu dem Absatz 4 der Führungsbohrung 2 innerhalb des Griffstückes 1 befindet und vor Beschädigungen geschützt ist. Zum Gebrauch der Vorrichtung wird die Schraube 17 entfernt und der Kolben 7 ist in der Führungsbohrung 2 frei verschiebbar.

Zum Induzieren eines nadelstichähnlichen Reizes wird die Vorrichtung mit ihrer Längsachse senkrecht zu der zu beaufschlagenden Gewebsoberfläche, z.B. der Haut, eines Probanden ausgerichtet, wobei der Kolben 7 in seine untere Endlage gleitet. Hierbei gelangt Luft durch die Durchgangsbohrung 6 des Deckels 3 in die Führungsbohrung. Anschließend wird die Spitze 15 der Nadel 14 auf die Gewebsoberfläche aufgesetzt und das Griffstück 1 in Richtung des Probanden verlagert, wobei der Kolben 7 in Richtung des Deckels 3 gleitet und die in der Führungsbohrung 2 vorhandene Luft durch die Durchgangsbohrung 6 im Deckel 3 entweicht. Aufgrund der definierten Masse des Kolbens 7 ist die Nadel 14 durch eine definierte, zur Masse des Kolbens 7 proportionale Kraft beaufschlagt, die derart gewählt ist, dass sie eine Schmerzempfindung bei völliger Abwesenheit von Schädigungen des Körpergewebes bei dem Probanden auslöst. Hierbei erfolgt durch die Spitze 15 der Nadel 14, deren Länge so gewählt ist, dass sie nicht in einem dicken Fettgewebe versinkt, eine Verschiebung des Körpergewebes von der Oberfläche in die Tiefe (Indentation) unter einer starken lokalen Scherung des Körpergewebes ohne eine Verletzung desselben. Aufgrund der definierten Masse des Kolbens 7 und der daraus resultierenden definierten Beaufschlagungskraft der Nadel 14 ist die Reizauslösung reproduzierbar. Da der Kolben 7 reibungsarm in der Führungsbohrung 2 gelagert ist, können die bei der Verschiebung wirksam werdenden Reibungskräfte bei einer Kräftebilanz vernachlässigt werden. Ebenso ist die Krafteinwirkung der Nadel 14 auf den Probanden unabhängig von der auf das Griffstück 1 ausgeübten Anpresskraft, da diese lediglich eine mehr oder weniger große Verschiebung des Kolbens 7 und damit der Nadel 14 bewirkt, die sich nicht auf die Reizauslösung auswirkt.

Um Veränderungen in der Schmerzempfindung eines Probanden bzw. Patienten einer reproduzierbaren quantitativen Beschreibung zugänglich zu machen, kommen mehrere im Wesentlichen identisch aufgebaute Vorrichtungen zum Einsatz, die sich lediglich durch die unterschiedlichen Massen ihrer Kolben 7 voneinander unterscheiden. Die Massen der Kolben 7 werden so gewählt, dass die erzeugten, die Nadel 14 beaufschlagenden Kräfte eine den Grundprinzipien der somatosensorischen Wahrnehmung entsprechende geometrische Reihe bilden und Bereiche unterhalb, um und oberhalb der Schmerzempfindungsschwelle umfassen, wobei die größte auf das Körpergewebe einwirkende Kraft eine solche Größe aufweist, dass in jedem Fall eine Verletzung der Haut des Probanden ausgeschlossen ist. Die Massen der Kolben 7 der Vorrichtungen werden beispielsweise so bemessen, dass Kräfte von 8, 16, 32, 64, 128, 256 und 512 mN auf die Haut des Probanden einwirken.

Zur Reinigung bzw. Desinfektion wird die Nadel 14 der Vorrichtung entnommen, indem der Kolben 7 durch Angreifen an der Ringnut 12 des Ansatzes 10 der Kappe 9 fixiert und die Nadel 14 aus der Führungshülse 13 gezogen wird. Nach dem Abnehmen des Deckels 3 von dem Griffstück 1 lässt sich der Kolben 7 aus der Führungsbohrung 2 entfernen.

Um eine möglichst reibungsarme, schmiermittelfreie Lagerung des Kolbens 7 zu realisieren, weist die Führungsbohrung 2 eine verhältnismäßig hohe Oberflächengüte, wie sie durch Hohnen oder Reiben gefertigt wird, auf und sowohl der Führungsring 8 als auch die Kappe 9 sind aus Polytetraflourethylen gefertigt. Damit die Vorrichtung im Rahmen bildgebender Verfahren der medizinischen Diagnostik ohne störende Auswirkungen zu verwenden ist, sind das Griffstück, der Kolben und die Nadel aus Aluminium, Messing oder einem nicht magnetischen Edelstahl gefertigt.

## Patentansprüche

1. Vorrichtung zur Induktion von nichtgewebsschädigenden Reizen zum Testen einer mechanisch induzierten Schmerzempfindung eines Probanden mit einem Griffstück (1), in dem eine von einer definierten Kraft beaufschlagte Nadel (14) mit abgeflachter Spitze (15) gleitbeweglich gelagert ist, **dadurch gekennzeichnet, dass** die Nadel (14) auswechselbar an einem in dem Griffstück (1) verschiebbar gelagerten Kolben (7) definierter Masse befestigt ist, wobei aus der Masse des Kolbens (7) die definierte Kraft resultiert.

2. Vorrichtung nach Anspruch 1, **dadurch gekennzeichnet, dass** die Nadel (14) an der Stirnseite einer Kappe (9) angeordnet ist, die an einer Stirnseite des Kolbens (7) zu dessen Gleitführung in einer zentrischen Führungsbohrung (2) in dem Griffstück (1) festgelegt ist.

3. Vorrichtung nach Anspruch 2, **dadurch gekennzeichnet, dass** die Nadel (14) in eine in der Kappe (9) befestigte Führungshülse (13) eingesetzt ist.

4. Vorrichtung nach Anspruch 2 oder 3, **dadurch gekennzeichnet, dass** die im Querschnitt zylindrische Kappe (9) einen Ansatz (10) aufweist, der aus einer stirnseitigen Öffnung des Griffstückes (1) nach außen ragt.

5. Vorrichtung nach Anspruch 4, **dadurch gekennzeichnet, dass** der Ansatz (10) mit einer Ringnut (12) versehen ist.

6. Vorrichtung nach Anspruch 3 oder 4, **dadurch gekennzeichnet, dass** der Kolben (7) unter Zwischenanordnung mindestens eines Führungsringes (8) in der Führungsbohrung (2) des Griffstückes (1) geführt ist.

7. Vorrichtung nach einem der Ansprüche 5 oder 6, **dadurch gekennzeichnet, dass** die Kappe (9) und/oder der Führungsring (8) aus Polytetrafluorethylen gefertigt ist.

8. Vorrichtung nach einem der Ansprüche 1 bis 6, **dadurch gekennzeichnet, dass** das Griffstück (1) auf der der Nadel (14) gegenüberliegenden Seite mit einem Deckel (3) verschlossen ist.

9. Vorrichtung nach Anspruch 6, **dadurch gekennzeichnet, dass** der Deckel (3) in das Griffstück (1) eingepresst oder eingeschraubt ist.

10. Vorrichtung nach Anspruch 7 oder 8, **dadurch gekennzeichnet, dass** der Deckel (3) eine konzentrisch zu dem Kolben (7) ausgerichtete Durchgangsbohrung (6) aufweist.

11. Vorrichtung nach einem der Ansprüche 6 bis 8, **dadurch gekennzeichnet, dass** in die dem Deckel (3) zugeordnete Stirnseite des Kolbens (7) eine Gewindebohrung (16) fluchtend zu der Durchgangsbohrung (6) des Deckels (3) eingelassen ist.

12. Vorrichtung nach einem der Ansprüche 2 bis 11, **dadurch gekennzeichnet, dass** die Führungsbohrung (2) des Griffstückes (1) stirnseitig einen verjüngenden Absatz (4) aufweist, der ein Austreten der Kappe (9) aus dem Griffstück (1) verhindert.

13. Vorrichtung nach Anspruch 12, **dadurch gekennzeichnet, dass** dem Absatz (4) innerhalb der Führungsbohrung (2) ein O-Ring (5) zugeordnet ist, der den Ansatz (10) der Kappe (9) mit Spiel umgreift.

14. Vorrichtung nach einem der Ansprüche 1 bis 13, **dadurch gekennzeichnet, dass** die Länge der Nadel (14) derart bemessen ist, dass sich ihr freies Ende innerhalb der Führungsbohrung (2) befindet, wenn der Kolben (7) an dem Deckel (3) anliegt.

15. Vorrichtung nach einem der Ansprüche 1 bis 14, **dadurch gekennzeichnet, dass** als Transportsicherung eine in die Durchgangsbohrung (6) des Deckels (3) eingesetzte Schraube (17) vorgesehen ist, die in die Gewindebohrung (16) des Kolbens (7) eingreift, um denselben an dem Deckel (3) festzulegen.

16. Vorrichtung nach einem der Ansprüche 1 bis 15, **dadurch gekennzeichnet, dass** das Griffstück (1), der Kolben (7) und/oder die Nadel (14) aus Aluminium, Messing oder einem nicht magnetischen Edelstahl gefertigt sind.

## Claims

1. Device for inducing non-tissue-damaging stimuli in order to test a mechanically induced sensation of pain in a test person, with a handle (1) in which a needle (14) with a flattened tip (15) is mounted so as to be able to slide when subjected to a defined force, **characterized in that** the needle (14) is secured exchangeably on a plunger (7) of defined mass mounted displaceably in the handle (1), wherein the defined force results from the mass of the plunger (7).

2. Device according to Claim 1, **characterized in that** the needle (14) is arranged on the end face of a cap (9), which is fixed on an end face of the plunger (7) for guiding the latter slidably in a central guide bore (2) in the handle (1).

3. Device according to Claim 2, **characterized in that** the needle (14) is fitted into a guide sleeve (13) secured in the cap (9).

4. Device according to Claim 2 or 3, **characterized in that** the cap (9) is of cylindrical cross section and has an attachment (10), which protrudes outwards from an opening in the end face of the handle (1).

5. Device according to Claim 4, **characterized in that** the attachment (10) is provided with an annular groove (12).

6. Device according to Claim 3 or 4, **characterized in that** the plunger (7) is guided in the guide bore (2) of the handle (1) with at least one guide ring (8) arranged therebetween.

7. Device according to either of Claims 5 and 6, **characterized in that** the cap (9) and/or the guide ring (8) is made from polytetrafluoroethylene.

8. Device according to one of Claims 1 to 6, **characterized in that** the handle (1), on the side opposite the needle (14), is closed with a cover (3).

9. Device according to Claim 6, **characterized in that** the cover (3) is pressed into or screwed into the handle (1).

10. Device according to Claim 7 or 8, **characterized in that** the cover (3) has a through-bore (6) oriented concentrically with respect to the plunger (7).

11. Device according to one of Claims 6 to 8, **characterized in that** a threaded bore (16), which is flush with the through-bore (6) of the cover (3), is let into the end face of the plunger (7) assigned to the cover (3).

12. Device according to one of Claims 2 to 11, **characterized in that** the guide bore (2) of the handle (1) has on its end face a tapering shoulder (4), which prevents an escape of the cap (9) from the handle (1).

13. Device according to Claim 12, **characterized in that** the shoulder (4) inside the guide bore (2) is assigned an O-ring (5), which engages with play around the attachment (10) of the cap (9).

14. Device according to one of Claims 1 to 13, **characterized in that** the length of the needle (14) is measured such that its free end is located inside the guide bore (2) when the plunger (7) bears on the cover (3).

15. Device according to one of Claims 1 to 14, **characterized in that** a screw (17) inserted into the through-bore (6) of the cover (3) is provided as a transport safety feature and engages in the threaded bore (16) of the plunger (7) in order to fix the latter on the cover (3).

16. Device according to one of Claims 1 to 15, **characterized in that** the handle (1), the plunger (7) and/or the needle (14) are made of aluminium, brass or a non-magnetic high-grade steel.

## Revendications

1. Dispositif servant à induire des stimuli n'entraînant pas de dommage tissulaire pour vérifier une sensation de douleur induite mécaniquement chez un sujet d'expérience, comportant une poignée (1) dans laquelle une aiguille (14) avec une pointe aplatie (15) soumise à une force définie est logée en mouvement coulissant, **caractérisé en ce que** l'aiguille (14) est fixée de façon remplaçable à un piston (7) de masse définie logé de façon coulissante dans la poignée (1), la force définie étant due à la masse du piston (7).

2. Dispositif selon la revendication 1, **caractérisé en ce que** l'aiguille (14) est disposée sur le côté frontal d'un capuchon (9), qui est fixé sur un côté frontal du piston (7) pour le guidage coulissant de celui-ci dans un alésage de guidage central (2) dans la poignée (1).

3. Dispositif selon la revendication 2, **caractérisé en ce que** l'aiguille (14) est insérée dans une douille de guidage (13) fixée dans le capuchon (9).

4. Dispositif selon la revendication 2 ou 3, **caractérisé en ce que** le capuchon (9) de section transversale cylindrique présente un bout (10), qui sort vers l'extérieur hors d'une ouverture frontale de la poignée (1).

5. Dispositif selon la revendication 4, **caractérisé en ce que** le bout (10) est doté d'une gorge annulaire (12).

6. Dispositif selon la revendication 3 ou 4, **caractérisé en ce que** le piston (7) est guidé dans l'alésage de guidage (2) de la poignée (1) avec interposition d'au moins une bague de guidage (8).

7. Dispositif selon l'une des revendications 5 ou 6, **caractérisé en ce que** le capuchon (9) et/ou la bague de guidage (8) est fabriqué en polytétrafluoroéthylène.

8. Dispositif selon l'une quelconque des revendications 1 à 6, **caractérisé en ce que** la poignée (1) est fermée avec un couvercle (3) sur le côté opposé à l'aiguille (14).

9. Dispositif selon la revendication 6, **caractérisé en ce que** le couvercle (3) est serti ou vissé dans la poignée (1).

10. Dispositif selon la revendication 7 ou 8, **caractérisé en ce que** le couvercle (3) présente un alésage de passage (6) orienté de façon concentrique au piston (7).

11. Dispositif selon l'une quelconque des revendications 6 à 8, **caractérisé en ce qu'**un alésage fileté (16) est pratiqué dans le coté frontal du piston (7) associé au couvercle (3) en alignement avec l'alésage de passage (6) du couvercle (3).

12. Dispositif selon l'une quelconque des revendications 2 à 11, **caractérisé en ce que** l'alésage de guidage (2) de la poignée (1) présente côté frontal un épaulement rétréci (4), qui empêche une sortie du capuchon (9) hors de la poignée (1).

13. Dispositif selon la revendication 12, **caractérisé en ce qu'**un joint torique (5) est associé à l'épaulement (4) à l'intérieur de l'alésage de guidage (2), et entoure avec du jeu le bout (10) du capuchon (9).

14. Dispositif selon l'une quelconque des revendications 1 à 13, **caractérisé en ce que** la longueur de l'aiguille (14) est dimensionnée d'une manière telle que son extrémité libre se trouve à l'intérieur de l'alésage de guidage (2), lorsque le piston (7) est appliqué contre le couvercle (3).

15. Dispositif selon l'une quelconque des revendications 1 à 14, **caractérisé en ce qu'**il est prévu comme sécurité de transport une vis (17) introduite dans l'alésage de passage (6) du couvercle (3), et qui s'engage dans l'alésage fileté (16) du piston (7) afin de caler celui-ci contre le couvercle (3).

16. Dispositif selon l'une quelconque des revendications 1 à 15, **caractérisé en ce que** la poignée (1), le piston (7) et/ou l'aiguille (14) sont fabriqués en aluminium, en laiton ou en un acier allié non magnétique.
